# EUROPEAN PATENT APPLICATION

(11) **EP 3 056 489 A1**
(43) Date of publication of application: **17.08.2016**
(21) Application number: 14845778.1
(22) Date of filing: 23.01.2014
(51) Int. Cl.: C07D 303/42, C07D 301/12

(54) **SYNTHESIS PROCESS FOR DIACETYL EPOXY GLYCERYL OLEATE**

(30) Priority: 23.09.2013 CN 201310441273
(71) Applicant: Guangzhou Hairma Chemical (GZ) Ltd., Guangzhou, Guangdong 510430 (CN)
(72) Inventor: LI, Daobin, Guangzhou Guangdong 510430 (CN); CAI, Qihong, Guangzhou Guangdong 510430 (CN); LIN, Chaojie, Guangzhou Guangdong 510430 (CN)
(74) Representative: Serjeants LLP
(86) International application number: PCT/CN2014/071177
(87) International publication number: WO 2015/039409

(57) **Abstract**

The present invention relates to a process for the synthesis of diacetyl epoxy glyceryl oleate, comprising the steps of: a) esterification of glycerol and oleic acid at 115-190°C for 1.5-4.5 hours in the presence of an esterification catalyst to obtain glyceryl monooleate; b) acetylation of glyceryl monooleate and an acetylating reagent at 90-160°C for 2-10 hours in the presence of an acetylation catalyst to obtain diacetyl glyceryl oleate; c) epoxidation of diacetyl glyceryl oleate and hydrogen peroxide at 60-80°C for 2-4 hours in the presence of an epoxidation catalyst and a weak acid to obtain diacetyl epoxy glyceryl oleate. The prepared diacetyl epoxy glyceryl oleate can be used as plasticizer in plastics, with the advantages of improved stability, good flowability, and high compatibility with plastics.

## Description

### Technical field

The present invention relates to a process for the synthesis of diacetyl epoxy glyceryl oleate.

### Background art

In recent years, plastics industries have been developed rapidly, which promotes economic growth of plastic auxiliaries industries, such as plasticizers, at a rate of an average 10.9% a year. Plasticizers, as functional products added to high molecular polymers to improve plasticity, workability and flexibility of materials, have been widely applied in various plastics products which are durable and easy to shape, such as toys, building materials, auto parts, electronic and medical device components, such as plasma bags, complete infusions, being species of plastics processing auxiliaries of maximum capability and consumption, about 60% of the total output of plastic auxiliaries. Plasticizers are modem plastics industry biggest auxiliary species, for Plastic auxiliaries 60% of total output. At present, there are up to 200 species of plasticizers in the world, each species exhibits respective physical and chemical performances, and different application fields.

Traditional plasticizers have mainly comprised phthalates containing phenyl ring structures, such as dioctyl phthalate (DOP) and dibutyl phthalate (DBP). As these plasticizers have been widely used in foods, medical industries, people are paying more and more attention to their toxicity. Foods may cause the phthalates in plastics products to dissolve out and penetrate into the foods during processing, heating, packaging, and filling, and thereby have potential toxicity to human bodies. Therefore, some countries have prohibited or restricted use of the plasticizers in food and medical fields. Recently, with the improvement of environmental protection awareness, plasticizers of plastics products in medical and food packages, commodities, and tops have higher requirements. It is inevitable to replace traditional plasticizer species with green and environmental friendly plasticizers. Epoxy plasticizers, as non-toxic environmental friendly plasticizers, become a focus of global study, mainly including epoxy oil and epoxy fatty acid esters, in which epoxidized soybean oil is the most important and common. It has excellent heat resistance, improved light resistance and low temperature flexibility, low volatility, and no toxicity, and thus is one of green plasticizers widely used in China. However, epoxidized soybean oil exhibits limited compatibility with PVC, and may be exuded from articles when it is used in a large amount (above 15%); when it is used as a stabilizer, color change will take place at high temperature to thereby impact the quality of articles. Therefore, it can be only used as an assistant plasticizer and assistant stabilizer, instead of primary plasticizer of PVC. Epoxy fatty acid esters have excellent lubrication property, compatibility and dispersity, in addition to the advantages of epoxidied oil. For instance, epoxy fatty acid methyl ester is a non-toxic plasticizer used in processing of plastics and rubbers, it has the advantages of excellent lubrication property, compatibility and dispersity, in addition to the advantages of high compatibility with products, low volatility, small migration, improved toughness of articles, non-toxicity, and good light and heat stability of epoxy plant oils as plasticizers and stabilizers. It can be added in an amount of 10-30% in processing of PVC articles.

It has been reported that fully acetylated glycerol monoester and fully acetylated glycerol monoester on 12-hydroxystearic acid have small molecular weight (about 500), higher boiling point (>300°C) and higher flash point (245°C), higher flowability and lower viscosity, and can be used as plasticizers of PVC. While epoxy oils have big molecular weight, high viscosity and poor flowability, however, since they contain epoxy group, they have good heat stability and high compatibility with plastics; glycerol triacetate is of small molecule weight, and has a viscosity less than epoxy oils. On the basis of their features and properties in structure, we hope to find epoxy acid esters plasticizers having plasticization effect similar to epoxy oils and lower molecular weight and improved flowability.

### Summary of the invention

The object of the present invention is to provide a process for the synthesis of diacetyl epoxy glyceryl oleate. The diacetyl epoxy glyceryl oleates prepared by this process are used as plasticizers in plastics, with the advantages of good stability, improved flowability and high compatibility with plastics.

To achieve the object, the present invention comprises the following technical solutions:
A process for the synthesis of diacetyl epoxy glyceryl oleate, comprising the steps of:
   a) esterification of glycerol and oleic acid at 115-190°C for 1.5-4.5 hours in the presence of an esterification catalyst to obtain glyceryl monooleate (A): 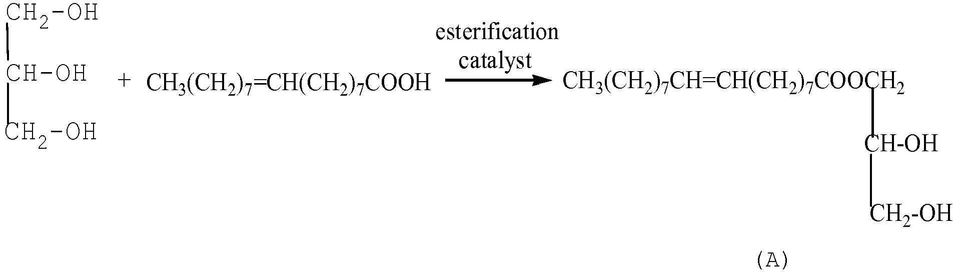
   b) acetylation of glyceryl monooleate (A) from step a) and an acetylating reagent at 90-160°C for 2-10 hours in the presence of an acetylation catalyst to obtain diacetyl glyceryl oleate (B); 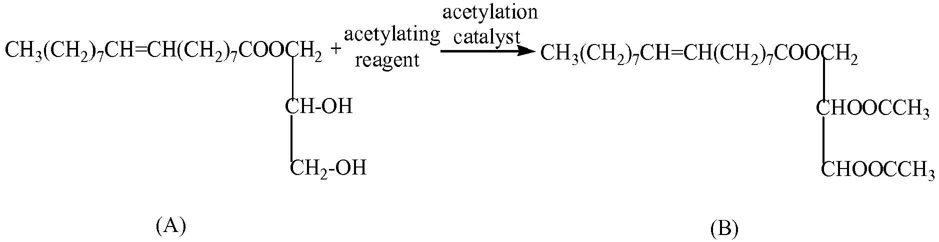
   c) epoxidation of diacetyl glyceryl oleate (B) obtained in step b) and hydrogen peroxide at 60-80°C for 2-4 hours in the presence of an epoxidation catalyst and a weak acid to obtain diacetyl epoxy glyceryl oleate (C);

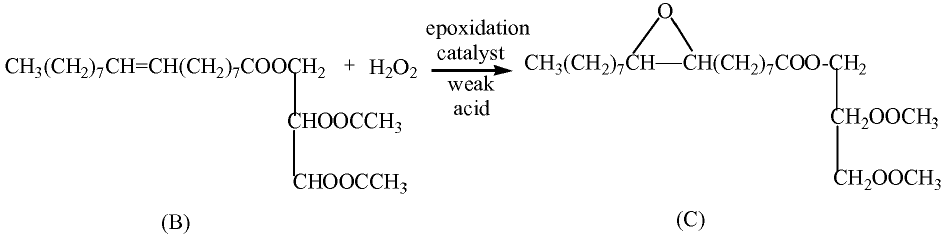

Preferably, in step a), the weight ratio of glycerol to oleic acid is 1:3.06-3.07.

Preferably, in step a), the esterification catalyst is concentrated sulfuric acid, concentrated phosphoric acid, potassium bisulfate, p-toluenesulfonic acid or [emin]BF4 ion liquid, and the amount of the esterification catalyst is 0.01-0.04% by weight of the esterification starting materials.

Preferably, in step b), the acetylating reagent is any one of acetyl chloride, glacial acetic acid, and acetic anhydride, and the amount of said acetylating reagent is 40-50% by weight of the amount of glyceryl monooleate.

Preferably, in step b), the acetylation catalyst is at least one of concentrated sulfuric acid, concentrated phosphoric acid and NaHSO4·H2O, and the amount of said acetylation catalyst is 0.3-0.6% by weight of the amount of glyceryl monooleate.

Preferably, a water-carrying agent is added to step b), the water-carrying agent is at least one of benzene, cyclohexane and hexane, and the amount of said water-carrying agent is 35-40% by weight of the amount of glyceryl monooleate.

Preferably, in step c), the epoxidation catalyst is concentrated phosphoric acid and/or concentrated sulfuric acid.

Preferably, in step c), the weak acid is formic acid and/or acetic acid.

Preferably, in step c), the concentration of hydrogen peroxide is 50%.

Preferably, in step c), the weight ratio of diacetyl glyceryl oleate, hydrogen peroxide, weak acid, and epoxidation catalyst is 1:0.4-0.7:0.04-0.08:0.001-0.003.

The advantageous effect of the present invention are: the diacetyl epoxy glyceryl oleates prepared by the process of the invention are used as plasticizers in plastics, with the advantages of plasticization effects similar to epoxy oils, and lower molecular weight, better stability and flowability, and higher compatibility with plastics than epoxy oils.

### Description of the drawings

Fig. 1 shows the infrared spectrum of glyceryl monooleate (A) according to the present invention;
Fig. 2 shows the infrared spectrum of diacetyl glyceryl oleate (B) according to the present invention;
Fig. 3 shows the infrared spectrum of the product diacetyl epoxy glyceryl oleate (C) according to the present invention.

### Embodiments

The embodiments of the present invention are further described as follows using examples. The present invention is not limited to the examples.

In the present invention, oleic acid and glycerol were used as starting materials to prepare diacetyl epoxy glyceryl oleate. The synthesis process comprises the following steps:
a) esterification of glycerol and oleic acid at 115-190°C for 1.5-4.5 hours to obtain glyceryl monooleate (A): 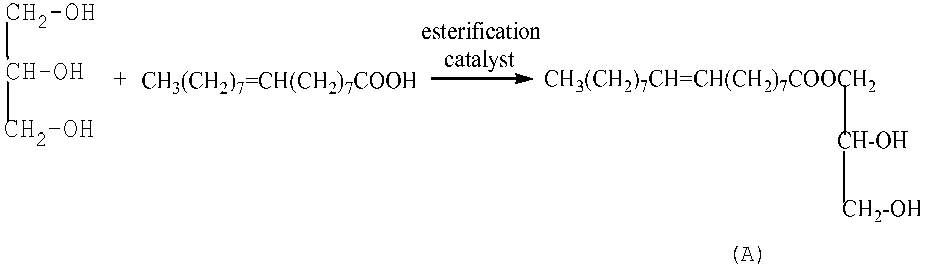
b) acetylation of glyceryl monooleate (A) from step a) and an acetylating reagent at 90-160°C for 2-10 hours to obtain diacetyl glyceryl oleate (B); 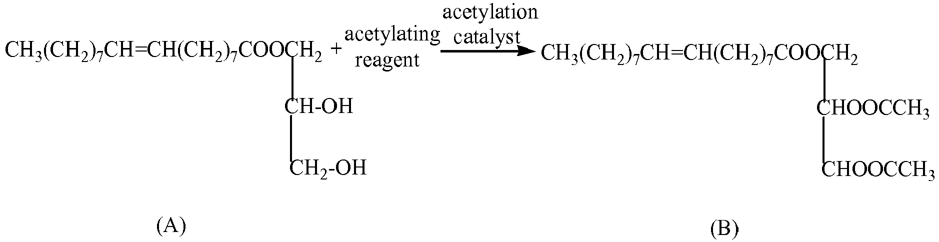
c) epoxidation of diacetyl glyceryl oleate (B) obtained in step b) and hydrogen peroxide at 60-80°C for 2-4 hours in the presence of an epoxidation catalyst to obtain diacetyl epoxy glyceryl oleate (C);

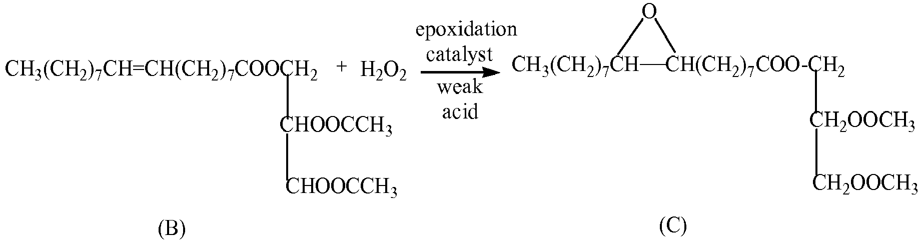

Wherein, in step a), the weight ratio of esterification starting materials glycerol to oleic acid was 1:3.06-3.07; the esterification catalyst was concentrated sulfuric acid, concentrated phosphoric acid, potassium bisulfate, p-toluenesulfonic acid or [emin]BF4 ion liquid, and the amount of the esterification catalyst was 0.01-0.04% by weight of the esterification starting materials.

In step b), the acetylating reagent was any one of acetyl chloride, glacial acetic acid, and acetic anhydride, and the amount of said acetylation reagent was 40-50% by weight of the amount of glyceryl monooleate; the acetylating reagent was at least one of concentrated sulfuric acid, concentrated phosphoric acid and NaHSO4·H2O, and the amount of said acetylation reagent was 0.3-0.6% by weight of the amount of glyceryl monooleate; a water-carrying agent was added to the acetylation reaction, the water-carrying agent was at least one of benzene, cyclohexane and hexane, and the amount of said water-carrying agent was 35-40% by weight of the amount of glyceryl monooleate.

In step c), the epoxidation catalyst was concentrated phosphoric acid and/or concentrated sulfuric acid; the weak acid was formic acid and/or acetic acid; and the weight ratio of diacetyl glyceryl oleate, hydrogen peroxide, weak acid, and epoxidation catalyst was 1:0.4-0.7:0.04-0.08:0.001-0.003.

### Example 1

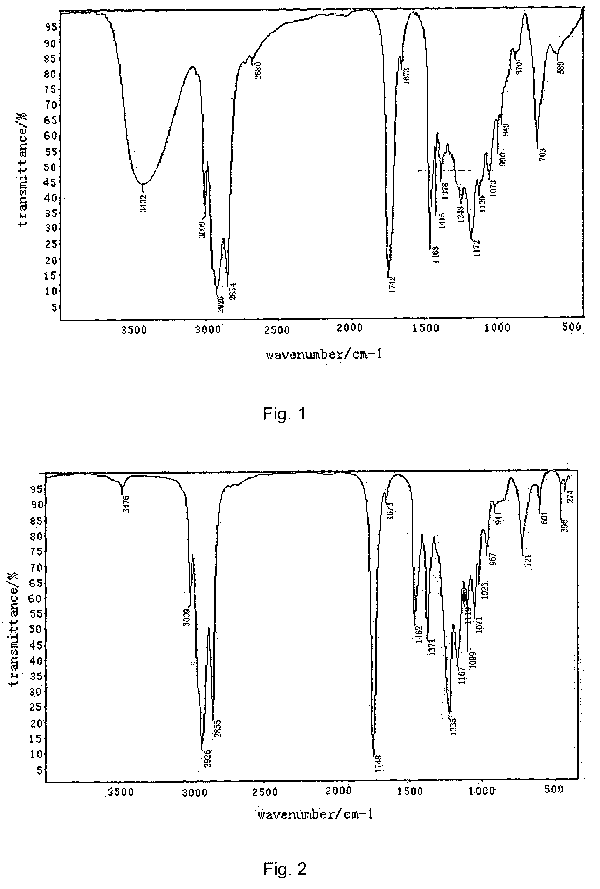

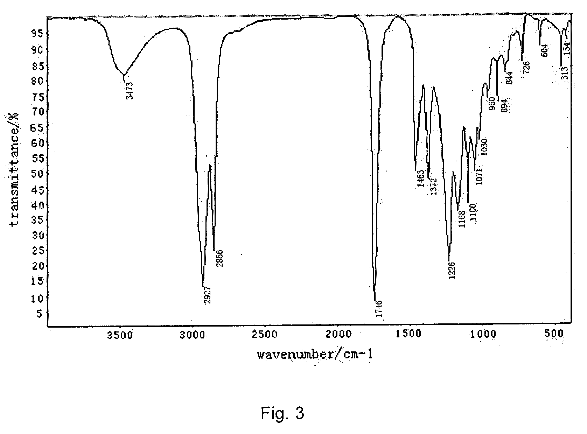

Diacetyl epoxy glyceryl oleate was prepared from oleic acid and glycerol by the following steps:
a) esterification: to a three-necked round bottom flask with a stirrer, a thermometer, and a reflux condenser were added 754 g of oleic acid and 240 g of glycerol successively. Introduced with nitrogen and heated with stirring. When the temperature reached 120°C, to the mixture was added 0.2 g of concentrated phosphoric acid catalyst, and allowed to esterify at 190°C for 3 hours, vacuumed for 25 hours until the acid value below 3, the reaction was stopped to obtain glyceryl monooleate. The results of the infrared measurement (IR(KBr)) of glyceryl monooleate were shown in Fig. 1: 3432cm⁻¹ broad peak for -OH peak, 3009cm⁻¹ for C-H stretching vibration absorption of unsaturated hydrocarbon; 2926cm⁻¹ for C-H asymmetric stretching vibration absorption of saturated hydrocarbon CH₂; 2854cm⁻¹ for C-H symmetric stretching vibration absorption of saturated hydrocarbon CH₂; 1742cm⁻¹ for C=O stretching vibration absorption of an ester; 1463cm⁻¹ for C-H asymmetric bending vibration of methyl group; 1378cm⁻¹ for C-H symmetric bending vibration of methyl group; 1172cm⁻¹ for C-O-C asymmetric stretching vibration; and 1120cm⁻¹ for C-O-C symmetric stretching vibration.
b) Acetylation: to a three-necked round bottom flask with a stirrer, a thermometer, a water-carrying device, and a reflux condenser were added 356 g of glyceryl monooleate obtained in step a), 160 g of glacial acetic acid as acetylating agent, 130 g of benzene as water-carrying agent, and 1.5 concentrated sulfuric acid as a catalyst, heated to 120°C and refluxed for 6 hours. The reaction was stopped when the outlet water quantity reached the theoretical quantity. Unreacted glacial acetic acid and benzene as water-carrying agent were extracted by reduced pressure. Black oily diacetyl glyceryl oleate was obtained. The results of the infrared measurement (IR(KBr)) of diacetyl glyceryl oleate were shown in Fig. 2: 3009cm⁻¹ for C-H stretching vibration absorption of unsaturated hydrocarbon; 2926cm⁻¹ for C-H asymmetric stretching vibration absorption of saturated hydrocarbon CH₂; 2855cm⁻¹ for C-H symmetric stretching vibration absorption of saturated hydrocarbon CH₂; 1748cm⁻¹ for C=O stretching vibration absorption of an ester; 1462cm⁻¹ for C-H asymmetric bending vibration of methyl group; 1371cm⁻¹ for C-H symmetric bending vibration of methyl group; 1167cm⁻¹ for C-O-C asymmetric stretching vibration; and 1119cm"1 for C-O-C symmetric stretching vibration.
c) Epoxidation: to a four-necked flask with a stirrer, a thermometer, a dropping funnel, and a reflux condenser were added 500 g of diacetyl glyceryl oleate obtained in step b), 30 g of 80-85% formic acid, and 0.7 g of concentrated sulfuric acid as catalyst successively, heated to 65°C, added dropwise slowly 250g of 50% hydrogen peroxide with stirring, heated to 80°C. The mixture was allowed to react for 4 hours. The lower acidic water was separated; the upper oil was washed with lye and water successively to neutrality, when the acid value was below 0.6, the resultant was distilled under reduced pressure to remove water and filtered to obtain light yellow oily diacetyl epoxy glyceryl oleate, with an epoxy value of 3.0, an acid value of 1.2, and an iodine value of 2.7. The results of the infrared measurement (IR(KBr)) of diacetyl epoxy glyceryl oleate were shown in Fig. 3: 2927cm⁻¹ for C-H asymmetric stretching vibration absorption of saturated hydrocarbon CH₂; 2856cm⁻¹ for C-H symmetric stretching vibration absorption of saturated hydrocarbon CH₂; 1746cm⁻¹ for C=O stretching vibration absorption of an ester; 1463cm⁻¹ for C-H asymmetric bending vibration of methyl group; 1372cm⁻¹ for C-H symmetric bending vibration of methyl group; 1168cm⁻¹ for C-O-C asymmetric stretching vibration; and 1110cm⁻¹ for C-O-C symmetric stretching vibration; 844cm⁻¹ for three-membered cycloether C-O-C asymmetric stretching vibration; and 726cm⁻¹ for methylene rocking vibration.

The examples described in the present invention are used only for describing the specific implementation of the process for preparing diacetyl epoxy glyceryl oleate from oleic acid and glycerol, not the limitation of the process for preparing diacetyl epoxy glyceryl oleate according to the present invention. Any modification and improvement to the process, and substitution and use of same or similar substances in patent scope or category all belong to the protection scope of the present invention.

## Claims

1. A process for the synthesis of diacetyl epoxy glyceryl oleate, comprising the steps of:
a) esterification of glycerol and oleic acid at 115-190°C for 1.5-4.5 hours in the presence of an esterification catalyst to obtain glyceryl monooleate (A): 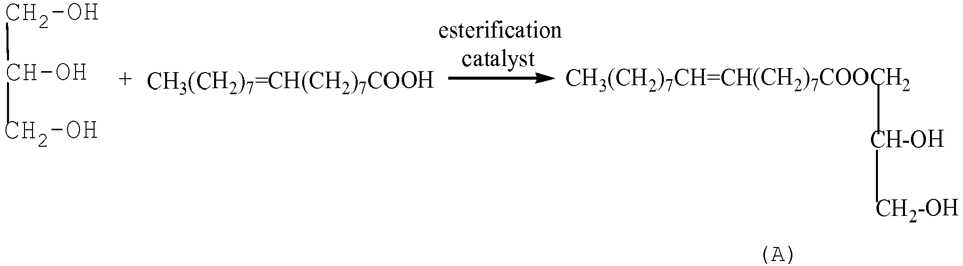
b) acetylation of glyceryl monooleate (A) from step a) and an acetylating reagent at 90-160°C for 2-10 hours in the presence of an acetylation catalyst to obtain diacetyl glyceryl oleate (B); 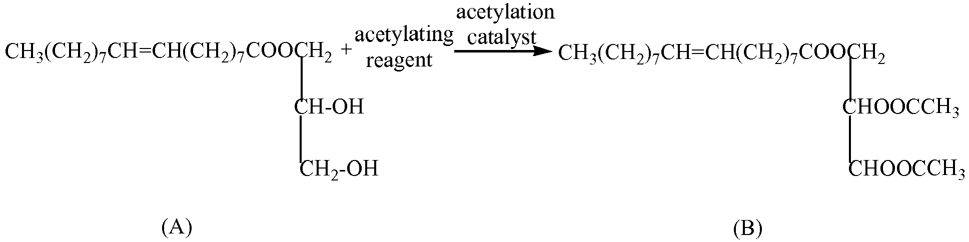
c) epoxidation of diacetyl glyceryl oleate (B) obtained in step b) and hydrogen peroxide at 60-80°C for 2-4 hours in the presence of an epoxidation catalyst and a weak acid to obtain diacetyl epoxy glyceryl oleate (C);

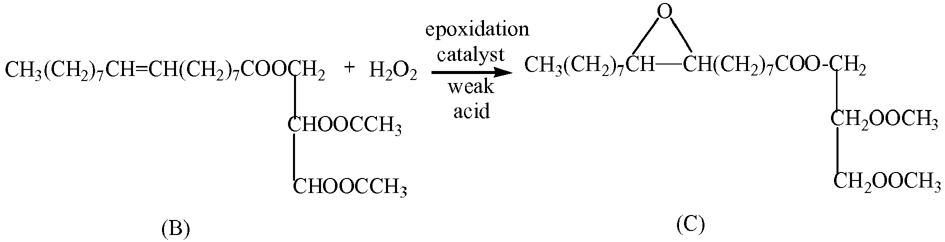

2. The process according to claim 1, **characterized in that** in step a), the weight ratio of glycerol to oleic acid is 1:3.06-3.07.

3. The process according to claim 1, **characterized in that** in step a), the esterification catalyst is concentrated sulfuric acid, concentrated phosphoric acid, potassium bisulfate, p-toluenesulfonic acid or [emin]BF4 ion liquid, and the amount of the esterification catalyst is 0.01-0.04% by weight of the esterification starting material.

4. The process according to claim 1, **characterized in that** in step b), the acetylating reagent is any one of acetyl chloride, glacial acetic acid, and acetic anhydride, and the amount of said acetylation reagent is 40-50% by weight of the amount of glyceryl monooleate.

5. The process according to claim 1, **characterized in that** in step b), the acetylation catalyst is at least one of concentrated sulfuric acid, concentrated phosphoric acid and NaHSO₄·H₂O, and the amount of said acetylation catalyst is 0.3-0.6% by weight of the amount of glyceryl monooleate.

6. The process according to claim 1, **characterized in that** a water-carrying agent is added to step b), the water-carrying agent is at least one of benzene, cyclohexane and hexane, and the amount of said water-carrying agent is 35-40% by weight of the amount of glyceryl monooleate.

7. The process according to claim 1, **characterized in that** in step c), the epoxidation catalyst is concentrated phosphoric acid and/or concentrated sulfuric acid.

8. The process according to claim 1, **characterized in that** in step c), the weak acid is formic acid and/or acetic acid.

9. The process according to claim 1, **characterized in that** in step c), the concentration of hydrogen peroxide is 50%.

10. The process according to claim 1, **characterized in that** in step c), the weight ratio of diacetyl glyceryl oleate, hydrogen peroxide, weak acid, and epoxidation catalyst is 1:0.4-0.7:0.04-0.08:0.001-0.003.
